Europäisches Patentamt

European Patent Office

Office européen des brevets

(19)

(11) Publication number : **0 662 327 A1**

## (12) EUROPEAN PATENT APPLICATION

(21) Application number : **95200006.5**

(22) Date of filing : **04.01.95**

(51) Int. Cl.⁶ : **A61K 38/16**

(30) Priority : **05.01.94 ES 9400017**

(43) Date of publication of application :
**12.07.95 Bulletin 95/28**

(84) Designated Contracting States :
**AT BE CH DE DK FR GB GR IE IT LI NL PT SE**

(71) Applicant : **INDUSTRIAL FARMACEUTICA Y DE ESPECIALIDADES, S.A.**
**Alameda de Urquijo, 20-27**
**E-48008 Bilbao (ES)**

(72) Inventor : **Palacios Pelaez, Ricardo**
**Avda. Moscatelar, 6-8,**
**Chalet Parcela C.**
**Madrid (ES)**
Inventor : **Martinez Garate, Alberto**
**José Miguel de Brandlaran, 11-4 Izda**
**Santurce (Vizcaya) (ES)**
Inventor : **Martinez Quesada, Jorge**
**Viuda de Epalza, 13-4 H**
**Bilbao (ES)**

(74) Representative : **Ungria Lopez, Javier et al**
**Avda. Ramon y Cajal, 78**
**E-28043 Madrid (ES)**

(54) **Use of self-incompatability receptor proteins (SLG and SRK) of plants for the treatment of allergic diseases.**

(57) The self-incompatibility receptor proteins (SLG and SRK) plants are useful in the treatment of allergic diseases by IgE. Likewise, they are useful to manufacture drugs used for said treatments.

The proteins can come from any plant, among which Brassica, Alnus glutinosa (alder), Parietaria judaica (pellitory), Populus alba (poplar), for example, can be cited.

Said proteins can totally or partially inhibit the IgE-allergen bond when tested "in vitro" in numerous systems, among which the mite of home dust, bee poison and pollen of Phleum pratense, Parietaria judaica, grass and Olea europea can be cited. Therefore, they are products useful in the treatment of allergic diseases.

EP 0 662 327 A1

TECHNICAL FIELD OF THE INVENTION

The present invention fits within the technical field of products of plant origin for the treatment of allergic diseaes mediated by IgE.

More particularly, the present invention refers to novel uses of the self-incompatibility receptors of plants (SLG § SRK) in the potential treatment of said diseases with high effectiveness based on their demonstrated capacity of interference of the IgE-allergen bond in vitro.

PRIOR ART OF THE INVENTION

Plants with flowers (angiosperms) are in the majority hermaphrodites (95%), in other words, they have in the same individuals male sex organs - pollens, produced by the anthers - and female sex organs, which correspond to the pistils. This fact together with the immobility of plants is a factor that predisposes self-fertilization, a phenomenon by which species would lose genetic variability that enables their survival. In order to avoid this, plants have a self-incompatibility mechanism based on stopping growth of the pollinic tube, a mechanism since 1977 is known to be widely distributed in angiosperms. ((1) Nettancourt D. Incompatibility in angiosperms. Springer-Verlag, New York (1977.)

Cross fertilization in these plants takes place when the grain of pollen of another individual of the same species reaches the top hole of the pistil called the stigma, where once the pollen is recognized, the development of the pollinic tube begins, which drops down the neck of the pistil (style) until it reaches the ovary located inside, where fertilization of the ovule will take place.

The development of molecular genetic techniques applied to the field of plant physiology has led to rather advanced knowledge of the self-incompatibility mechanisms, basically in two plant species: cabbage (Brassica oleracea) and an ornamental tobacco (Nicotina alata.) Specifically, the Adrianne Clarke group of the University of Melbourne (Australia) identified in 1986 the genes responsible for self-incompatibility (called S genes) in Nicotina alata ((2) Anderson M.A. Cornish E.C., Mav, S.L., Williams E.G., Hogart R. Cloning of cDNA for a stylar glycoprotein associated with expression of self-incompatibility in Nicotiana alata. Nature, 321: 38-44 (1986)) and in 1985, the Mike and June Nasrallah team of Cornell University (United States) identified the first S genes in Brassica oleracea. ((3) Nasrallah J.B., Kao, T.H. Goldberg M.L., Nasrallah M.E., A cDNA clone encoding an S-locus-specific glycoprotein from Brassica oleracea. Nature, 318, 263-267 (1985.))

Brassica seems to have a more developed self-incompatibility model than Nicotiana and has been the center of most studies carried out since then. Thus, the amino acid sequence of the glycoproteins expressed by the S genes has beeen characterized ((4) Nasrallah J.B., Kao T.H., Chen C.H., Goldberg M.L., Nasrallah M.R. Amino acid sequence of glycoproteins encoded by three alleles of the S locus of Brassica oleracea. Nature, 326; 617-619 ((1987), which are the receptor molecules that are going to recognize the grain of pollen, and that are going to give rise to neutralization thereof in the event that it is from the same individual. One type of these proteins has been previously called SLSG (S-locus specific glycoproteins) and lately as SLG.) They are polymorphic and encoded by different alleles, having molecular wieghts between 57 and 65 kilodaltons ((5) Nasrallah J.B., Nasrallah M.E., The molecular genetics of self-incompatibility in Brasicca. Ann. Rev. Genet, 23: 121-139 (1989.))

The different alleles have well conserved regions alternated with hypervariable regions ((6) Nasrallah J.B., S.M., Nasrahhal M.E., Self-incompatibility genes of Brassica oleracea: Expression, isolation and structure. Proc. Natl. Acad. Sci. USA. 85, 5551-5555 (1988)), which indicates a similarity with the structure of the genes of antibodies or of the larger complex of histocompatibility that determines the recognition between one's own and that of another. In this case, it is the mechanism inversely, to distinguish one's own, which is rejected, and to accept the other pollen.

Regarding SLG of Brassica it is also known that they are synthesized in the stigmas ((7) Nasrallah, J.B., Doney R.C. and Nasrallah M.E., Biosynthesis of glycoproteins involved in the pollen-stigma interaction of incompatibility in developing flowers of Briassica oleracea L. Planta 165, 100-107 (1985)), and that they are accumulated in their papillary cells where the barrier for the development of incompatible pollen is constituted ((8) Kandasamt M.K., Paolillo D.J., Faraday C.D., Nasrallah J.B., Nasrallah M.E. The S locus specific glycoproteins of Brassica accumulate in the cell wall of developing stigma papillae. Dev. Biol. 134: 462-472 (1989)). The self-immunity response is manifested in one or two days prior to anthesis (or the release of pollen from the floral anthers) and it has been verified that during these days it is precisely when the accumulation and synthesis of said proteins are produced in said proteins in the stigma ((7) and (9) Riberts I.N., Stead A.D., Ockendon D.J., Dickinson H.G. A glycoprotein associated with the acquisition of the self-incompatibility system by maturing stigmas of Brassica oleracea. Planta 146, 179-183 (1979.))

The degree of knowledge of these self-incompatibility receptor proteins (SLSG) is so advanced that not only is the nucleotide sequence of the genes that they express (SGL genes) known ((10) Scott C.P., Gates P.J., Gatehouse J.A., Boulter D., Croy R.D. A cDNA encoding and S-locus specific glycoprotein from Brassica oleracea plants containing the $S_5$ self-incompatibility allele. Mol. Gen. Genet. 220: 409-413 (1990)) and the amino acid and glucide composition thereof known ((11) Takayama S., Isoga A., Tsukamoto C., Ueda Y, Hinata K. Structure of Carbohydrate chains of S-glycoproteins in Brassica campestris self-incompatibility locus. Nature, 326: 102-104 (1987)), but monoclonal antibodies against it has also been produced whereby the synthesis thereof in the cytoplasm of papillary cells with subsequent incorporation into the walls of said cells has been confirmed by immunocytochemical techniques ((8) and (12) Roberts I.N., Harrod G. and Dickinson H.G. Pollen-stigma interactions in Brassica oleracea I. Ultrastructure and physiology of the stigmatic papillary cells. J. Cell. Sci. 66: 241-253 (1984.) Despite this, the molecular mechanism of the neutralization of the self-pollen through the self-compatibility system represented by SLG proteins has not yet been clarified, just as Dumas et al. observe ((13) Dumas C., Gaude T., Heizmann P. Rougier, La Recherche, 13:576-578 (1993)), although it has been pointed out that the self-incompatibility response operates at the cell-cell interaction level between those of the grain of pollen and the papillary ones with which it has contact in the stigma.

A second type of proteins, SRK, are also receptors and responsible for self-incompatibility in sporophytic control systems (such as those that operate in Brassica and Alnus) are also being the object of extensive research (in the field of molecular genetics applied to farm research) in the last few years. Hence, it is known that it is a kinase enzyme that, therefore, increases phosphorylation of the proteins that interact with it leading to a change in the cellular physiology. It is expressed in the papillary cells of the stiga at the same time as the SLG. It has the same range of molecular weights and forms a complex with them in the membrane of the papillary cells in order to interact on the whole with the proteins of the pollen, on which the recognition that leads to its accpetance or rejection takes place ((14) Nasrallah J.B., Stein J.C., Kandasamy M.K., Nasrallah M.E. Science, 266: 1505-1508 (1994.))

It is also known that this type of protein SRK (S-receptor kinase) are encoded by a different gene than the SLG, that has an amino acid sequence (and of nucleotides in the gene thereof) perfectly explained, which is highly homologus to that of the SLG proteins in the area corresponding to the extracellular domain thereof, also having a great deal of similarity with the immunoglobulin domain present in the superfamily members of immunoglo bulins ((15) Glavin T.L., Goring D.R., Schafer U., Rothstein S.J., Mol. Gen. 244; 630-637(1994.))

Although in the flowers of other plants the self-incompatibility system has not been so thoroughly studied as in Brassica, proteins of the style associated with the pollen recognition process and the subsequent development thereof in the tube in species such as: Prunus avium (cherry) ((16) Mau S.L., Raff J., Clarke A.E., Isolation and partial characterization of component Prunus avium styles, including an S-allele-associated antigenic glycoprotein. Planta 156: 505-516 (1982)), Lycopersicon perunavium (tomato) ((17) Mau S.L., Williams E.G., Atkinson A., Anderson M.A., Cornish M.C., Grego B., Simpson R.J., Kheyr-Pour A., Clarke A.E., Style proteins of a wild tomato (Lycopersicon peruvianum) associated with expression of self-incompatibility. Planta 169: 184-191 (1986)), Nicotiana alata (ornamental tobaco) ((18) Jahnen W., Batterham M.P., Clarke A.E., Moritz R.L., Simpson R.J. Indentification, isolation and N-terminal sequencing of style glycoproteins associated with self-incompatibility in Nicotiana alata. Planta Cell., I.: 493-499 (1989)); (19) Kuroda S., Ishihara K., Sakiyama F. Use of SMART System for micropreparation of a plant protein. Science Tools 36:8-9 (1992)) and Petunia hybrida ((20) Linskens H.F., Incompatibility in Petunia. Proc. R. Soc. Lond. 188:299-311 (1975); and (21) Broothaerts W.J., Van Laere A., Witters R., Preavy G., Decock B., Van Damme J., Vnedrig J.C. Purification and N-terminal sequencing of style glycoproteins associated with self-incompatibility in Petunia hybrida. Plant. Molecular Biology 14: 93-102 (1989)), have been characterized. As a whole the SLG proteins of these plants have common aspects such as their basic nature and a molecular weight between 24 and 65 kDa (21.) Besides, in petunia it has been described that the inhibition of the pollinic tube is done all along the style, these proteins being found not only in the stigma but also in the intracellular matrix of the style ((20) and (21.)) The SLG protein of Nicotiana has been purified to homogeneity and in a physiologically active state from a few styles, by means applying the SMART chromatographic system (19.)

As it has been demonstated in the above bibliographic references, research on SLG § SRK receptor proteins has always been approached in the plant physiology and molecular genetics fields, taking into consideration their use in matters regarding plant fertilization and hybridization, but in no case, under the use that is presented in the following specification.

Up to the present date, the use of said proteins for the treatment of allergic diseases has not been patented, published or reported - nor even suggested. A use that the applicant bases on the two following postulates:

1) Patients with allergies or hypersensitization, have as a common pattern, an increase in their serum indexes of the immunoglobulin E antibody (IgE.) This IgE is specific of the allergen to which the individual is sensitized and has a great affinity to the same. Normally, there are two cellular types that have receptors for the Fc fragment of IgE: basophils in the blood and abundant mastocytes in the connective tissue.

The IgE, once fixed by the Fc fragment to the previous cellular types dimerously bridge the allergen and cause the release of the histamine stored in the cytoplasmic granules and other preformed mediators towards the contiguous tissues causing the corresponding allergic setting, depending on the location of the mastocytes in the sensitized tissue (rhinitis, conjunctivitis, asthma, etc.)

Presently, allergy due to immediate hypersensitization is treated well by immunotheraphy, or else by pharmacotherapy. In the first case, which is a causal treatment, the gradual incorporation of the proteins that cause the allergy in the patient progressively desensitize him by means of the stimulus in the production of specific "blocking" IgG antibodies and other cellular events.

The drugs used the most in (symptomatic) treatment of allergy are: antihistamines (analogous competitors of receptors for histamine), membrane stabilizers (chromoglycate and nedocromil), corticosteroids, β- agonists, antichlonergics and broncodilators ((22) Holgate S.T., Church M.K., Allergy, Gower Medical Publishing, London, New York (1993.))

2) During the last two years, there has been an extraordinary interest in the study of profilins, that have been defined as pan-allergens, since they are very omnipresent proteins and it has been demonstrated by the Dutch group of Aalberse ((23) Van Ree R., Voitenko V., Van Leeunen W.A., Aalberse R.C. Profilin is a cross-reactive allergen in pollen and vegetable foods. Int. Arch. Allergy Immunol. 98: 97-104 (1992)) and the Austrian group of Valenta ((24) Vallier P., Dechamp C., Valenta R., Vial O., Deviller P. Purification and characterization of an allergen from celery immunochemically related to an allergen present in several other plant species. Identification as a profilin. Clin. and Exper. Allergy, 22: 774-782 (1992)) that are important allergens in pollens and foods of numerous species studies, being responsible for the cross reactivity thereof. Even the existence of similar structures in honey and hymenoptera venom has been suspected ((25) Aalberse R.C., Koshte V., Clemens J.G.J. Immunoglobulin E. antibodies that cross-react with vegetable foods, pollen and hymenoptera venom. J. Allergy Clinn Immunol. 68: 356-364 (1981)). It is known that profilins are proteins that control the polymerization of actin in eukaryotic cells ((26) Aderem A., Signal transduction and the actin cytoskeleton: the roles of Marcks and profilin. TIBS 17: 438-443 (1992)) and due to their function in the actin cytoskeleton they play a fundamental role in the development of the pollinic tube ((27) Valenta R. et al. Profilins constitute a novel family of functional plant pan-allergens. J. Exper. Med. 175: 377-385 (1992.))

In contrast to the present state of the art cited above, the present invention proposes the uses of SLG § SRK proteins as anti-idiotypes or factors of interference in the bond to the immunoglobulins, considering that, when the allergens come into contact with the mucous membrane (nasal, bronchial or ocular) of the target organs of patients with allergy, in conditions of humidity and temperature similar to those of pistils, they can be neutralized by said SLG § SRK previously introduced into the body, given that the pollen profilin (an important panallergen) is neutralized in the stigmas (or styles) of the flowers by the cited receptor proteins.

In accordance with the above, the applicant proposes the use of SLG and SLK proteins in patients affected by allergy due to immediate hypersensitization, as a possible treatment for these pathologies.

## BRIEF DESCRIPTION OF THE FIGURES

Figure 1 RAST-inhibition of D. pteronyssinus with Alnus extract.
Figure 2 RAST-inhibition of Phleum pratense with Alnus extract.
Figure 3 RAST-inhibition of Parietaria judaica with Alnus extract.
Figure 4 RAST-inhibiton of a mixture of grasses with Alnus extract.
Figure 5 RAST-inhibition of Olea europea with Alnus extract.
Figure 6 RAST-inhibition of bee venom with Alnus extract.

## DETAILED DESCRIPTION OF THE INVENTION

Just as it is indicated in its title, the present invention refers to the use of self-incompatibility receptor proteins (SLG and SRK ) of plants for the treatment of allergic diseases mediated by IgE.

The raw material from which the isolation of the SLG and SRK proteins is carried out, constitutes pistols or female organs of plants with flowers (angiosperms.) These must be found in a specific state of maturation: the one corresponding to one or two days before anthesis. Isolation can be done by two alternative ways: biochemistry or genetics.

a) BIOCHEMISTRY: Extraction is done in an aqueous medium with magnetic stirring, that can be reinforced by using a more drastic homogenization system with grinding by shears. The hydrophobicity of the medium can be increased by adding surface active agents at a low concentration. The proteins solubilized in the aqueous medium are separated from the rest of the solid plant structure by means of centrifugation, followed by dialysis of the supernatant that contains them by means of tangential ultrafiltration, or else dialysis by passive diffusion. After dialysis, the extract is clarified by filtration, so that the raw extract of the pistil containing the SLG and SRK proteins in solution is obtained. The preparation may be stored in a stable manner by freezing or lyophilization.

The subsequent purification of the SLG proteins can be done by chromatography in a Sepharose concanavaline A column (specific for glycoproteins) or else by ion exchange chromatography in a cation exchanger with Mono-S filler (SMART system.)

b) GENETICS: In the specific case of Brassica, upon knowing the sequence of the genes that enclode SLG and SRK proteins, the first convenient oligonucleutides can be selected, to carry out the amplification of the gene by the PCR technique (Polymerase chain reaction) with subsequent expression in E. coli type microorganism transforming it with the resulting recombinant plasmide, obtaining said SLG and SRK proteins, carrying out first of all a comparative study of the recombinant proteins with the native ones to ensure their homology.

The SLG and SRK proteins extracted from stigmas show in SDS-PAGE electrophoresis (electrophoresis in polyacrylamide gel in the presence of sodium sulfate dodecyl) a complex scheme with several species of close molecular weights, differing in approximately 2000 daltons and with an average molecular weight of between 57 and 65 kDa. This polymorphism can be assumed given that it reflects the variability that the specific recognition of pollen requires.

In its moment of highest expression it constitutes 5% of the total soluble proteins synthesized in the stigma of the pistil.

The SLG have 5 to 7 lateral oligosaccharide chains in the N-terminal region of the protein. It has been determined that the oligosaccharides have a neutral sugar composition of xylose, fuchose and mannose in a proportion of 4.1:9:12.1 mol/mol of protein. The glycosidic bonds comprise the amide group of asparagine residue with anomeric N-acetylglucosamine carbon. The glucidic part of the molecule is not responsible for the specificity in the pollen-stigma interaction.

The SLG proteins have an average number of 436 amino acids and a basic isoelectric point around 8.6, while SRK have 462 amino acids, with regions of high homology with regard to SLG.

The applicant has used the SLG and SRK proteins contained in an purified extract to inhibit the RAST (Radio allergo sorbent test) reaction that constitutes an in vitro model of the allergic reaction and therefore results predictive of the operative mechanisms in patients with allergy.

The RAST technique was designed ((28) Ceska M., Lundquist U. A new and simple radioimmunoassay method for the determination of IgE. Immunochemistry 9:102-105 1972) for the in vitro diagnosis of type I allergic diseases (immediate hypersensitization) which are the ones mediated by the IgE type antibodies. Given that the demonstrated base of this allergy is the bond of the allergenic protein to the Fab fragments of its specific IgE, that are bonded by the Fc portion to the membrane of mastocytes and basophiles, with the subsequent setting off of cellular degranulation, as a consequence of the bridging of two Ige molecules, which gives rise to the release of mediators that cause the allergic symptomatology, a system was thought up, according to which, the allergens couple to a paper disk (by means of the chemical activation reaction) which was placed in the bottom of a tube. Upon adding the serum of a patient sensitized to an allergen in question, his specific Ige , by the Fab portion thereof, are fixed to the allergenic proteins of the disk. These IgE molecules are then displayed by adding human anti-IgE antibodies, which have a chemically conjugated enzyme, in such a way that the subsequent addition of a colorless substrate gives rise to the appearance of an easily quantifiable colored product.

It has been unmistakably demonstrated that there is an excellent correlation between the diagnosis in vitro by RAST nad the clinical skin test of patients with allergy ((28) Fadal R.G., Nalebuff D.J., in "RAST" in Clinical Allergy, Year Book, Medical Publishers, Chicago, 1981)), which means that the in vitro model is highly analogous and representative of what happens in the setting off of the allergic pathology.

On this basic technique, Yman et al. ((30) Yman L., Ponterious G., Brandt R. RAST-bases allergen assay mthods. In: Rigamy, A.P., Hennessen W., Perkins F.T. (eds) Developing Biological Standars. Basel: S. Karger Verlag, 29: 151-165, 1975) projected a variant consisting of adding the allergen in liquid phase to compete with the allergens of the disk by the serumal IgE, in such a way that the new technique (called RAST-inhibition) serves to evaluate the strength (affinity) of the allergic proteins added in liquid phase.

Within this technical framework and based on the hypothesis that the SLG and SRK proteins are capable of neutralizing the allergens of pollen and all those analogous structures - presumibly by previous fixation - the applicant carried out an entire series of tests in which the capacity of interference of said proteins isolated from pistils of different plants was tested, upon the IgE-allergen bond, in RAST-inhibition experiments, using different allergen-positive serum systems.

Given that, as it has been commented on above, the in vitro model of RAST agress with what happens in vivo, the RAST inhibition by addition of protein molecules in liquid phase that are unmistakably preventing the bonding of the immunoglobulin E to the allergen will probably be predietive of that in vivo its inclusion in the cellular environment of mastocytes and basophiles will likewise prevent access of the allergens to IgE that cover up the cellular membranes, whereby the setting off of the allergic pathology will be inhibited.

Specifically, proteins extracted from:

a) Immature influorescences of Alnus glutinosa (Alder)

b) Immature influorescences of Parietaria judaica (Pellitory)

c) Pistils of Tulipa sp (tulips)

d) Pistils of Populus alba (Poplar) have been used.

Each one of these cases is analyzed in detail in the following paragraphs:

a) The Alnus glutinosa (alder) is a plant with which more studies have been carried out. The Alnus influorescence is comprised of the pistil in more than 90% of its structure. The SLG-SRK proteins thereof were co-isolated by means of aqueous extraction obtaining a raw extract that was colorimetrically quantified and characterized by SDS-PAGE. Two extractions were made, in aqueous and slightly hydrophobic solvent (with Triton X-100.) Using both extracts at two different concentrations, the RAST to Parietaria judaica (pellitory) pollen, Olea europea (olive) pollen, mixture of grass pollens, Dermatophagoides pteronyssinus (home dust mite) and Apis mellifera (bee) poison was significantly inhibited. In all cases, a RAST-inhibition test was comparably carried out with each allergen at different concentrations as a comparative reference. Enriching the extract by precipitation with ammonium sulfate, the level of inhibition was increased. In all the allergenic systems, at the maximum concentration of SLG and SRK extract, similar levels of inhibition which were located between 50 and 70 % were found.

The extract with Triton-X inhibited to the largest degree the RAST to Olea europea pollen and Parietaria judaica pollen, while the aqueous extract showed similar levels of inhibition for grasses and bee poison.

These results are indicative of a certain hydrophobicity of SLG and SRK proteins, logical considering their function as cellular receptors.

b) With the proteins of Parietaria judaica pistils inhibition was obtained in the two allergenic systems tested. It inhibited up to high levels (80-90%) RAST to Parietaria judaica pollen and also to a lesser degree the RAST to another mite of home dust, Dermatophagoides farinae. The aqueous extract, as well as the one obtained with detergent (Triton X-100) showed an inhibitory power.

c) Tulip flowers have some very large pistils, however, inhibition with their protein extracts was not obtained, possibly due to the difficulty of obtaining plants in an expression period of self-incompatibility receptor proteins.

d) Using extracts of Populus alba (poplar) pistils all the allergenic systems tested have been inhibited: Helianthus annuss (sunflower) pollen, Parietaria judaica pollen, Lolium perenne (olive) pollen, mixtures of grasses and home dust mite, Dermatophagoides pteronyssinus. In all cases the RAST was inhibited at levels that varied from 44 to 73%, depending on the allergenic system and the concentration of pistil extract used.

Finally, it is noteworthy that with all the floral systems studied, the presence of proteins in the extract in the area of molecular weights of 45 to 65 Kda of SDS-PAGE gel compatible with SLS proteins as well as with SRK proteins, was verified.

## EMBODIMENTS OF THE INVENTION

The present invention is additionally illustrated by means of the following Examples, which do not seek to be restrictive of its scope which is defined solely and exclusively by the attached set of claims.

## Example 1: Isolation of the SLG-SRK complex from Alnus glutinosa

Immature female influorescences of Alnus glutinosa (Alder) of a stage prior to anthesis were used as raw material. The absence of other parts of plants or pollens was tested microscopically.

40 g. of said material (previously frozen to -20º C) were extracted by means of suspension in 200 ml. of ultra-purified water. The homogenization was done in a VIRTI-SHEAR-25 apparatus (shear homogenizer) at 25,000 rpm adding 50% of the volume of balls of glass of 400-600 μm to facilitate the breaking of the cell walls.

The separation of the solubilized proteins from the rest of the solid structure was done by centrifugation at 5,000 rpm for 30 minutes. The supernatant of centrifugation was dialysed against extrapurified water by tangential ultrafiltration in the Pellicon (Millipore) system with a cutting membrane of 5,000 daltons. The entire process was carried out at 4-8º C. In this way the aqueous extract which was characterized and used directly (after filtration through a membrane of a pore of 0.22 μ, or else stored in a lyophilized state until use thereof, was obtained.

The sediment from the cited centrifugation was subjected to extraction once again, this time in coconut liquid with Triton X-100 0.1% in a volume of 200 ml by magnetic stirring (11,000 rpm) for 48 hours at 4º C. Afterwards, the steps of centrifugation, ultrafiltration, filtration and lyophilization were carried out, under the same conditions as those described for the aqueous extract.

Example 2: Characterization of the Alnus glutinosa extract obtained in Example 1

The protein content of the Alnus glutinosa pistil extract was determined by the Bradford method (known commercially as BIO-RAD), in accordance with the following protocol:

Protein determination by the BIO-RAD method. Microassay procedure

It is based on the method designed by Bradford, based on lthe displacement of the maximum absorption wave length that Coomassie G-250 blue of 465 yo 595 nm undergoes when it is bonded to the proteins.

Preparation of the Standard:

From the commercial solution (BIO-RAD II) of BSA or MERCK albumin fraction, at a concentration of 1498 μg/ml which will be called Solution A, 7 new solutions (B, C, D, E, F, G, H) of which the last 5 (D-H) were used to construct the gauge line were prepared.

The solutions were formed in the following manner:

Solution A (1498 μg/ml) :          Defreeze an aliquot of the Commercial Standard
Solution B (150 μg/ml) :           0.1 ml. Solvent A + 0.9 ml $H_2O$ or SSFF
Solution C (75 μg/ml) :            1 ml. Solution B + 1 ml $H_2O$ or SSFF
Solution D (37.5 μg/ml) :          2 ml. Solvent C + 2 ml $H_2O$ or SSFF
Solution E (18.8 μg/ml) :          2 ml. Solvent D + 2 ml $H_2O$ or SSFF
Solution F (9.4 μg/ml) :           2 ml. Solution E + 2 ml $H_2O$ or SSFF
Solution G (4.7 ug/ml) :           2 ml. Solution G + 2 ml $H_2O$ or SSFF

Procedure:

A duplicate of 0.8 ml of the solutions D-E-F-G-H and a target ($H_2O$ or SSFF) was taken and 0.2 ml. of concentrated reagent were added to it, after stirring the mixture, the absorbancy at 595 nm from 5-60 nm. The absorbancy at 595 nm against the protein concentration was represented. The test was valid, since the correlation coefficient was equal or larger than 0.98.

Results:

By means of the described technique, a protein content of 5.4% was determined (with regard to the total lyophilized mass) for the aqueous extract and 9.1 % for the extract obtained with Triton X-100.

The characterization of the molecular weight of the proteins obtained was done by SDS-PAGE (electrophoresis on polyacrylamide gel in the presence of sodium sulfate dodecyl) in accordance with the method described hereinafter.

It was done basically by the Laemmli method, with slight, proper modifications and those designed by the manufacturers of the electrophoresis (horizontal) equipment used: Mini-Protean II (Bio-Rad.)

The buffers and solutions used were the following:

a. BIS-ACRYLAMIDE SOLUTION
Acrylamide                       87.6 g (29.2 g/100 ml)
N-N'-bis-methylene-acrylamide    2.4 g (0.8 g/100 ml)
Distilled water                  300 ml
Filtered and stored at 4º C in the dark

b. 1.5 M Tris-HCl pH = 8.8 (Running)
Tris base          27.23 g (18.15 g/100 ml)
Distilled water    150 ml
The pH was adjusted to 8.8 with HCl 1N and it was stored at 4º C until it was used.

c. 0.5 M Tris-HCl pH = 6.8 (Stacking)

Tris base 6 g.

Distilled water 100 ml

The pH was adjusted to 6.8 with HCl 1 N and it was stored at 4º C.

d. SDS SOLUTION 10 %

SDS 10 g.

Distilled water 100 ml

e. SAMPLE BUFFER

Distilled water 4.0 ml

0.5 M Tris-HCl pH = 6.8 1.0 ml

Glycerol 0.8 ml

SDS 10% 1.6 ml

2-B-nercaptoethanol 0.4 ml

Bromophenol blue 0.5 % 0.2 ml

The samples were heated at 100° C for 5 minutes.

f. ELECTRODE BUFFER pH = 8.3 (5X)

Tris base 9 g (15 g/l)

Glycine 43.2 g (72 g/l)

SDS 3.0 g (5 g/l)

Distilled water 600 ml

It was stored at 4º C and was heated to 37º C when precipitation took place.

60 ml. of stock 5X buffer were diluted with 240 ml of distilled water.

g. COOMASSIE BLUE SOLUTION (Stain)

Coomassie Blue R-250 1 g.

Methanol 400 ml

Acetic acid 100 ml

Distilled water 500 ml

Staining time: 30 minutes

h. DECOLORING SOLUTION

Methanol 400 ml

Acetic acid 100 ml

Distilled water 500 ml

Discoloring time: 1-3 hours

Method:

1. MOUNTING OF THE PLATES

 The instructions in the Mini-protean II dual slab cell catalogue were followed.

2. PREPARATION OF THE SEPARATING GEL (% of ACRYLAMIDE 12.5%)

```
Distilled water                   3.1 ml

1.5 M Tris-HCl pH = 8.8           2.5 ml (0.376 M)

SDS 10%                           100 µl (0.1 %)

Bis/acrylamide solution 4.3 ml (9.68% ACA; 0.26% BIS)

-------------------- degassing 10' --------------- -----------

Ammonium persulfate (fresh)       75 µl (30 mg/ml)

Temed                             6 µl
```

Amount per plate: 3.2 ml (polymerization time 1 h) INTERPHASE: 40 µl of SDS 0.1 % per each side of the plate

3. PREPARATION OF THE PREPARING GEL (% ACRYLAMIDE 4%)

```
Distilled water                    6.1 ml
0.5 M Tris-HCl pH 6.8              2.5 ml
SDS 10%                           100 µl

Bis-acrylamide solution           1.3 ml (3.79% ACA)
------------------- degassing 10' -----------------------
Ammonium persulfate (fresh)        75 µl
Temed                              10 µl
```

## 4. ELECTROPHORETIC CONDITIONS
Voltage: 200 volts
Time: 45 minutes

### Determination of molecular weights

It was done by constructing a gauge line, representing the mobility (in nm) shown by a series of proteins against the logarithm of their molecular weight (known.) Mobility was measured from the beginning of the separating gel to the middle point of the band. Once the correlation between both variables was shown (r>0.90), the molecular weight of the problem proteins or polypeptides was determined from their mobilities. The proteins used as standards were those of the BIO-RAD kit for low molecular weights (Ref. 161-0304.)

| Protein | Molecular weight (daltons) |
|---|---|
| Phosphorylase b | 97,400 |
| Bovine serum albumin | 66,200 |
| Egg albumin | 42,699 |
| Carbonic anhydrase | 31,000 |
| Trypsin inhibitor | 21,500 |
| Lysozyme | 14,400 |

### Results

In the corresponding electrophoretic scheme, it was observed that the Alnus pistil extract gave rise to an intense broad band in the area of 60 kDa of molecular weights, which is consistent with the polydisperse character of the SLG § SRK proteins. This band implies more than 90% of the proteins of the extract.

### EXAMPLE 3: Study of RAST-inhibition by SLG-SRK proteins

The capacity of SLG-SRK protector proteins to be used as the factor of interference of IgE was demonstrated by the RAST-inhibition technique. Said tests were carried out in accordance with the protocol described hereinafter:

### ELISA-inhibition with solid phase of cellulose disks (RAST-inhibition)

NOTE: To make the allergen solutions in free phase, the following incubation buffer was used.

9

Incubation buffer

| | |
|---|---|
| NaCl | 9 g |
| $Na_2HPO_4 . 2H_2O$ | 7.2 g |
| $NaH_2PO_4 . H_2O$ | 1.3 g |
| Human albumin 20% | 15 ml |
| Sodium azide | 0.5 ml |

Distilled water up to 1 liter

1. Different allergen concentrations were prepared in incubation buffer, according to the scheme (allergen in free phase: inhibitor)

   (a) : 10 mg/ml (based on dry weight of lyophilized material)

   (b) : 1 mg/ml

   (c) : 0.1 mg/ml

   (d) : 0.01 mg/ml

2. The allergen disks (solid phase) were placed in tubes, drying them well, by suction with a pipette in a vacuum.

3. 50 µl of positive serums and of each one of the allergen concentrations were added.

4. The tubes were closed up and incubated for 3 h. at room temperature.

5. Each tube was washed three times, with the washing solution of the Phadezym RAST tracer kit.

6. 50 µl of conjugate (β-galactosidase)anti Ige) enzyme) were added to each one of the disks (Phadezym RAST tracer.)

7. The tubes were closed up and incubated all night at 4º C.

8. They were washed 3 times just like in point 5.

9. 200 µl of development solution (Phadezym RAST tracer) were added. 200 µl of development solution were placed in two other tubes, without disks or allergen in free phase, or serum to carry out the reading targets.

10. The tubes were closed up and incubated exactly 2 hours at 37º C.

11. 1 ml. of stop solution* (Phadezym RAST tracer) was added.

12. The absorbancy at 420 nm was measured, adjusting to zero with the targets.

Results:

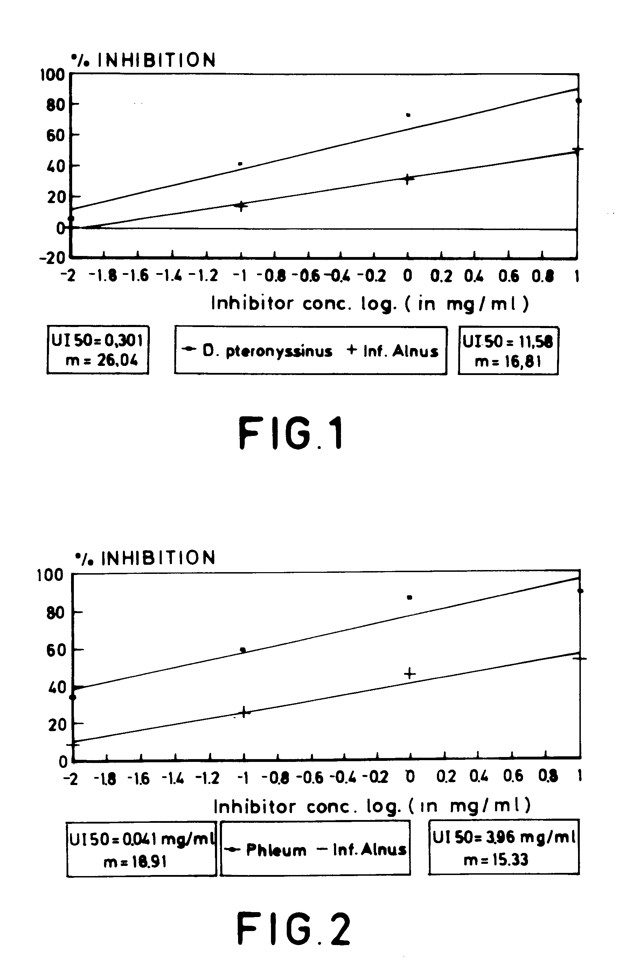

Some of the most representative results obtained by means of the described technique are presented in figures 1 to 6 and are commented on hereinafter:

Inhibition was carried out with aqueous Alnus glutinose extract, containing SLG and SRK proteins at different concentrations in three allergenic systems: mite D. pteronyssium, grass pollen Phleum pratense and underbrush pollen Parietaria judaica. Upon testing several concentrations of allergen and of Alnus, the inhibition lines were constructed and in this way, their two basic comparative parameters were evaluated: the gradient of the line (m) that gives an indication of the homology of the compared systems and the $UI_{50}$ value which, defined as the concentration of allergen in liquid phase that produces RAST-inhibition of 50%, provides information about the affinity (or strength) of each one of the antigen-antibody systems.

In the D. pteronyssinus system (figure 1), the Alnus extract produced inhibitions up to 50.7% (16 10 mg/ml), as to 82% of self-inhibition of D. pteronyssinus extract at this same concentration. As is observed in the figure, the gradients of the lines are similar, and the Alnus extract inhibits with a strength factor 38 times lower than that of D. pteronyssinus (calcuated by the quotient of the respective $UI_{50}$.)

The RAST-inhibtion to Phleum pratense pollen (figure 2) showed even greater parallelism between the lines of the homologus extract and the Alnus extract. The latter produced a maximum inhibition of 53.1 % (as to 88.3% of Phleum) and a relative strength 96 times lower.

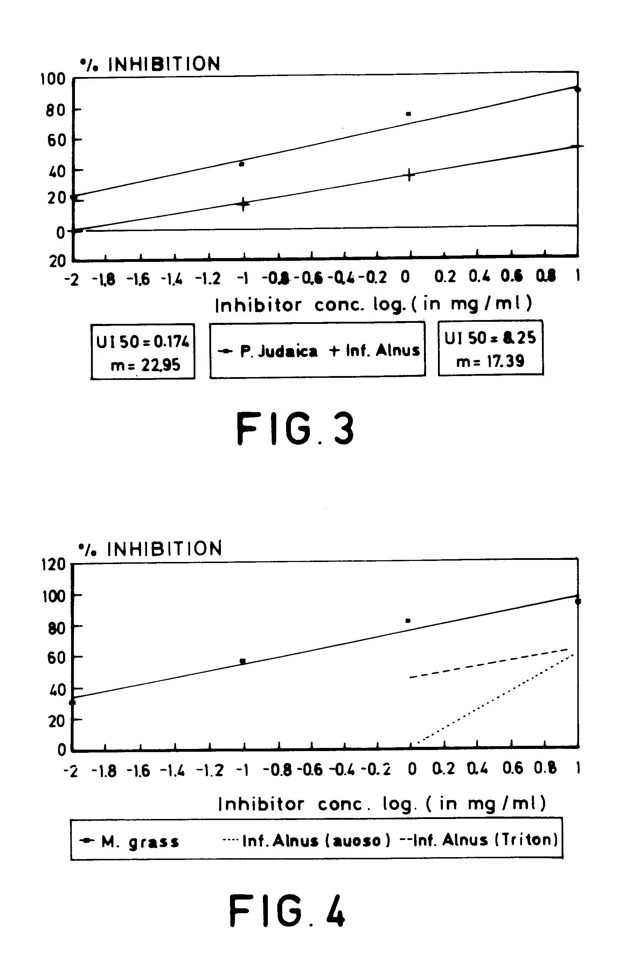

The line of RAST-inhibition to Parietaria judaica pollen (figure 3) caused by Alnus influorescence extract (pistil in more than 90%) was also parallel to that caused by the extract of the pollen itself, reaching 51.8 % inhibition at the maximum concentration used (10 mg/ml), while that of Parietaria was 87.6 %. The $UI_{50}$ quotient indicated a strength (affinity) 47 times lower in the Alnus extract.

In another series of experiments (figures 4-6) the inhibition of Alnus extracts was tested, aqueous as well as slightly hydrophobic ones (with Triton X-100) at two fixed concentrations 10 and 1 mg/ml, in three allergenic systems: mixture of grass pollens, Olea europea (olive) pollen and bee poision.

The RAST-inhibition to grass pollen is shown in figure 4. With both Alnus extracts a maximum inhibition

* Stop solution: 4.2 g. of sodium carbonate in 100 ml. of distilled water.

of 64.7 and 63.0 % was obtained, the one of the extract obtained with detergent (Triton X-100) being higher while the maximum produced by the pollen extract itself was 93.8%.

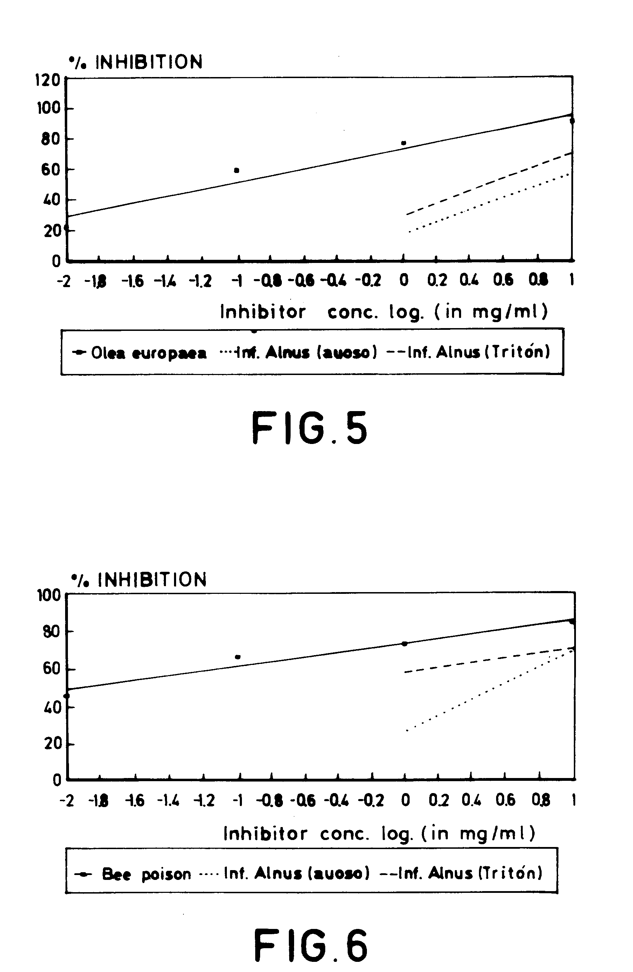

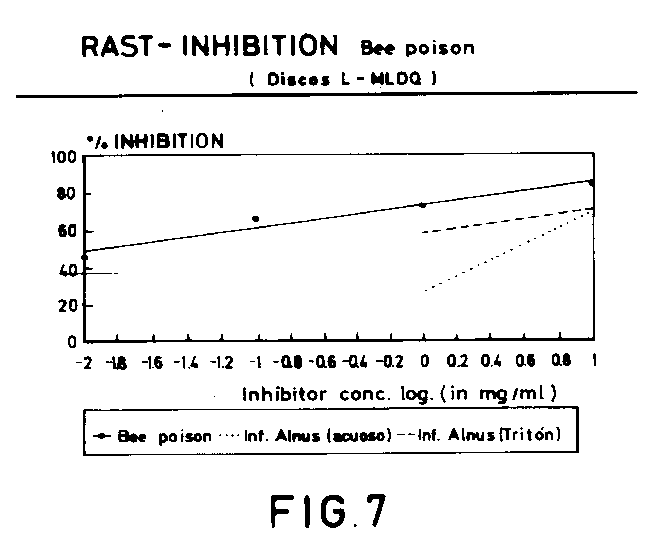

In the Olea pollen system (figure 5) the inhibition caused by the Alnus extract obtained with detergent was 71.4% at 10 mg/ml as to 55.4% of the aqueous extract and 91.5% of the Olea extract itself at the same concentrations.

A system so different as the bee poison one was also inhibited (figure 6), reaching inhibition maximums (similar) of 70.4 and 69.6% for the Alnus extracts, with detergent and aqueous respectively and 83.7% with the homologus extract, in other words, bee poison extract. At the concentration of 1 mg/ml once again the hydrophobic extract inhibits more, which ratifies once again that inhibitory proteins (SLG and SRK) have a slightly hydrophobic nature. They can be extracted with aqueous solvents or by adding detergents at low concentrations.

## Claims

1.- Use of self-incompatibility receptor proteins (SLG and SRK) extracted from the pistils of the flowers of plants angiosperms, in a stage of maturation corresponding to one or several days before anthesis, for the manufacture of drugs for the treatment of allergic diseases mediated by IgE.

2.- Use according to claim 1 characterized in that the cited proteins can also be obtained by Genetic Engineering techniques.

3.- Use according to any of the above claims characterized in that the cited protein belongs to the Brassica plant.

4.- Use according to any of the above claims characterized in that the cited protein belongs to the Alnus glutinosa (alder) plant.

5.- Use according to any of the above claims characterized in that the cited protein belongs to the Parietaria judaica (pellitory) plant.

6.- Use according to any of the above claims characterized in that the cited protein belongs to the Populus alba (poplar) plant.

7.- Use according to claim 5, characterized in that the Alnus glutinosa protein totally or partially inhibits the allergic reaction against Dermatophagoides pteronyssinus (home dust mite) in vitro.

8.- Use according to claim 5, characterized in that the Alnus glutinosa protein totally or partially inhibits the allergic reaction against Phleum pratense pollen in vitro.

9.- Use according to claim 5, characterized in that the Alnus glutinosa protein totally or partially inhibits the allergic reaction against Parietaria judaica pollen in vitro.

10.- Use according to claim 5, characterized in that the Alnus glutinosa protein totally or partially inhibits the allergic reaction against mixed grass pollens in vitro.

11.- Use according to claim 5, characterized in that the Alnus glutinosa protein totally or partially inhibits the allergic reaction against Olea europea pollen in vitro.

12.- Use according to claim 5 characterized in that the Alnus glutinosa protein totally or partially inhibits the allergic reaction against Apis mellifera (bee) poison in vitro.

EP 0 662 327 A1

FIG.1

FIG.2

FIG. 3

FIG. 4

**FIG.5**

**FIG.6**

FIG.7

**European Patent Office**

**EUROPEAN SEARCH REPORT**

Application Number

EP 95 20 0006

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| A | EP-A-0 519 869 (CIBA-GEIGY AG) --- | | A61K38/16 |
| A | EP-A-0 436 467 (CIBA-GEIGX AG) ----- | | |

TECHNICAL FIELDS SEARCHED (Int.Cl.6)

C07K

The present search report has been drawn up for all claims

| Place of search | Date of completion of the search | Examiner |
|---|---|---|
| THE HAGUE | 18 April 1995 | Rempp, G |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)